# EUROPEAN PATENT APPLICATION

(11) **EP 1 728 852 A1**
(43) Date of publication of application: **06.12.2006**
(21) Application number: 05011607.8
(22) Date of filing: 30.05.2005
(51) Int. Cl.: C12M 1/04

(54) **Fermenter system for biotechnical processes**

(71) Applicant: Chiron Behring GmbH & Co. KG, 35006 Marburg (DE)
(72) Inventor: Müller, Günther, 35510 Butzbach-Kirch-Göns (DE); Zimmermann, Achim, 35043 Marburg (DE); Vorlop, Jürgen, 35041 Marburg (DE)
(74) Representative: UEXKÜLL & STOLBERG

(57) **Abstract**

A fermenter for use in biotechnical processes in particular for culturing cells is disclosed. The object, to provide a fermenter vessel (1) the inner volume of which can be optically monitored and which fermenter vessel can also be efficiently sterilized is achieved by a fermenter vessel (1) having an outside wall (2) enclosing a vessel volume with a view port (7) being provided in the outside wall (2) for inspecting processes within the vessel volume, the view port (7) having a transparent member (8) wherein the transparent member (8) is provided with a heating device.

## Description

The present invention relates to a fermenter system for use in biotechnical processes in particular for culturing cells.

Biotechnical processes for the aerobic or anaerobic culturing of animal or microbial cells are usually carried out in bioreactors, often designated as fermenters. Here, the choice of the specific bioreactor is crucial for the efficiency of the process. Fermenters should provide for optimal conditions in view of organism growth and metabolic activity. They can be purchased in comparatively small laboratory scales of 1 to 10 liters as well as in industrial scales of up to 16.000 liters or more. The exact size of the fermenter depends on the yearly demand of product, the process in question and the mode of operation.

The term "fermenter" or "fermenter system" refers generally to a device which can be used for the aerobic or anaerobic culturing of cells. Such system may comprise one or more fermenter vessels which can be sealed in a manner so that the content of the vessel is seperated from the environment outside the system, thereby avoiding contamination of a certain cell culture grown in the fermenter system. The vessel comprises several inlets or outlets leading to the vessel which are used to connect pipes and tubing for the controlled introduction and/or removal of liquids or gases, such as media components or oxygen. These tubes and pipes connected to the vessel are also part of the fermenter system. Usually, these parts are cleaned and sterilized in a single step together with the vessel.

The most widely used type of a fermenter is a stirred tank vessel. This type of reactor usually consists of a cylindrical vessel of stainless steel which is closed at the top and into which various armatures like valves and pipes (e.g. for measuring probes or other ancillary equipment) are fitted.

Sterility of the fermenter system represents a key requirement for its successful operation. Particularly, in the context of the production of substances for pharmaceutical use (such as vaccines or active agents), any contamination of the cell culture in the fermenter has to be excluded. Therefore, the entire fermenter system including the ancillary equipment and the culture medium are sterilized prior to their use. However, known fermenters share certain constructive deficiencies which can lead to insufficient sterilization of the fermenter system prior to use which in turn can be the reason for contamination. Alternatively, contamination of the cell culture in the fermenter might occur as a consequence from processing steps during operation of the system.

In this connection, deadlegs are a common source of contamination in currently used fermenter systems. Deadlegs generally refer to parts of a tube or pipe or parts of a more complex conduction assembly, such as plumbing or ductwork, which are not completely flooded by the liquid or gas flowing through it. These sites are not properly reached during sterilization of the fermenter and residual culture media from an earlier production run may be trapped in these regions which in turn can cause contamination of the culture in a subsequent fermentation using this system. Especially the region of the valves in the tubing can often not be sterilized sufficiently. Therefore, there is the need to provide a fermenter system having valves which are free of deadlegs in order to ensure proper sterilization.

In biotechnical production processes it is often necessary to monitor the process by optically observing the broth within the closed vessel. Therefore, view ports are installed in the vessel wall which have a transparent member, usually a glass window, through which a user can look inside the vessel. Due to condensate on the transparent window the view through the window can be impeded. Here, it is known from the prior art to mount a wiper inside the vessel which is actuated from outside to clean the window. But it has turned out that the feed-through by means of which the movement of the wiper is effected is a reason of contamination since an efficient sterilization of the feed-through is usually difficult to achieve.

Thus, there is a need to provide a fermenter vessel the inner volume of which can be optically monitored and which fermenter vessel can also be efficiently sterilized.

Bioreactors for the culturing of cells need a stirrer for the homogeneous distribution of cells, nutrients, gases and heat. In conventional fermenter systems, the stirrer shaft is directly driven from outside the vessel. Due to this construction, contaminations are likely to occur over the rotating double mechanical seal. In the prior art, sterile steam condensate between the two ring pairs has been used in order to avoid contamination of the fermentation vessel. However, it has been shown that this method is also not suitable to exclude the risk of contamination. This results in the need for a fermenter vessel with an improved stirrer system which does not impede the sterilization of the fermenter.

During the sterilization of a fermenter system, steam is lead through the vessels and the tubing which results in a considerable increase in temperature of the material of the fermenter system. Especially the vessel wall and the tubing expand due to the increasing temperature. This expansion leads to tension and stresses in the material which can result in fractures and cracks. In turn, contaminations can occur in these fractures and cracks which can hardly be elimated just by leading steam through the tubings of the fermenter system. Thus, there is a need for a fermenter system in which the risk of forming cracks and fractures during sterilization is reduced.

During cell culturing processes, it is often preferred to transfer the culture broth from one vessel to another in order to increase the number of cells. Cell culture medium in batch processes only has the ability to generate at the end of one passage the 10-fold cell number compared to the initial cell number. Therefore, experts in the field of cell culturing produce a high number of cells in a first fermenter vessel. When a certain cell density is reached, a part of the cell culture to a further fermenter vessel with fresh culture medium. Such transfer is usually repeated several times, whereas the volume of the fermenter vessels increases with each transfer step. The volumetric scale up is normally done by a factor between 3 and 10. In the prior art, transfer is faciliated by use of pumps which forces the liquid to flow from one vessel in the direction of the feed vessel. However, such transfer leads to foaming which can damage the cells in the culture by generating shear stress. Thus, there is also the need to provide a fermenter system by use of which foam production and shear stress are avoided.

Based on the prior art, it is therefore the object of the present invention to provide a fermenter vessel and a fermenter system, respectively, which overcomes the above mentioned drawbacks.

According to a first aspect of the present invention, this object is achieved by a fermenter vessel having an outside wall enclosing a vessel volume with a view port being provided in the outside wall for inspecting processes within the vessel volume, the view port having a transparent member wherein the transparent member is provided with a heating device.

Due to the heating device condensate which has been formed on the inner surface of the transparent member can be removed without the need for a mechanical feedthrough through the vessel wall. This reduces the risk for contaminations in the fermenter system.

In a preferred embodiment, the heating device comprises a heating wire formed on the surface of the transparent member. This has the advantage that the surface of the transparent member can be efficiently heated up by a small amount of heating energy. In another embodiment, the heating device comprises an inner heating wire which is incorporated in the transparent member, leading also to an efficient heat up of the transparent member.

Alternatively or additionally a heating lamp arranged outside the vessel volume can be provided as a heating device to heat up the transparent member. This setup has the advantage that it is not necessary to modify the transparent member, and common view ports can be used which allows to retrofit an already installed view port.

According to another aspect of the present invention, the above object is achieved by a fermenter vessel having an outside wall enclosing a vessel volume with a stirring element arranged inside the vessel volume and with driving means arranged outside the vessel for rotationally driving the stirring element with the stirring element being provided with a first magnetic element and the driving means being provided with a second magnetic element wherein the first magnetic element and the second magnetic element are coupled via magnetic force.

Due to the magnetic coupling the risk of contaminations in the fermenter system is strongly reduced compared to the prior art where a mechanical coupling by a shaft going through the vessel wall was used. The magnetic coupling allows for a complete separation between the stirrer element inside the vessel and the driving means outside the vessel.

In a preferred embodiment, the stirring element comprises a stirrer shaft with the first magnetic element being mounted on the stirrer shaft. Furthermore, the driving means comprise a driving shaft with the second magnetic element being mounted on the driving shaft.

In order to achieve a good magnetic coupling between the magnetic elements, it is preferred that the vessel has a recess in the outside wall, the recess having an annular wall projecting into the vessel volume wherein the second magnetic element is arranged inside the recess. Here, the first magnetic element is arranged adjacent to the annular wall.

The above object is according to a third aspect of the present invention achieved by a fermenter vessel having an outside wall enclosing a vessel volume, the wall comprising an inlet for introducing or extracting a gas or liquid into or from the vessel volume, and a connection tube to a supply device for the gas or liquid wherein the connection tube comprises a first section extending along a first axis, a second section connected to the first section and extending along a second axis, the second axis being essentially perpendicular to the first axis, and a third section connected to the second section and extending along a third axis, the third axis being parallel and spaced apart to the first axis.

The loop formed by the first, second and third section can be bent along the sections when the sections expand as a result of an increase in temperature of the material during sterilization. This reduces the tensions in the tubing an thus the risk of crack formation. In a preferred embodiment a junction is provided in the second section, wherein the junction comprises an inlet valve. By this constructive means, the inlet valve is protected from tension and stress which arises during sterilization of the system. Furthermore, by means of the three sections, it is possible to scavenge the connection between the inlet valve and the vessel. Thus the tubing between the inlet valve and the vessel does not represent a deadleg.

According to another aspect of the present invention, a fermenter system is provided with a vessel enclosing a vessel volume and with a tubing for distributing liquids in the fermenter system wherein the tubing comprises a switching valve, the switching valve having a valve body, a plurality of outlet studs, a passage tube passing through the valve body from a first end to a second end, a plurality of bores in the valve body and a plurality of valve elements inserted into the bores wherein the first end and the second end are communicating with a liquid supply, wherein the bores are communicating with the outlet studs and wherein the bores intersect the passage tube.

Due to the direct connection between the passage tube and the bores which is achieved by the intersection between the bores and the passage tube the valve according to the present invention has no sections which can not be reached during sterilization with steam which is lead through the passage tube. In this connection, it has to be mentioned that an intersection in the sense of the present invention is already present, if part of the wall of the passage tube is intersected by the bores. The opening of the passage tube to the bores is directly closed by the valve elements and no space remains which is not scavenged when steam is lead through the tubing.

According to another aspect of the present invention, a fermenter system is provided which comprises a first fermenter vessel, a second fermenter vessel and a transfer tube connecting the first vessel and the second vessel, wherein the transfer tube is connected to the bottom section of the first vessel and to the bottom section of the second vessel.

By means of the transfer tube, it is possible that a transfer of broth from the first vessel to the second vessel takes place when the pressure is increased in the first vessel. In this case, the increased pressure in the first vessel forces part of the broth to move through the transfer tube into the second vessel. Here, pumps can be omitted and the foam producing effect of the pumps is eliminated.

In a preferred embodiment, a transfer valve is provided in the transfer tube which allows for the control of the flow between the vessels. Furthermore, it is preferred to provide a pressure inlet in the wall of the first vessel which allows to introduce a pressured medium, preferrably pressured air, to increase the pressure inside the first vessel.

The above mentioned aspects relating to fermenter vessels and systems, respectively, can also be applied in each possible combination with each other.

In the following, preferred embodiments of the present invention are described referring to the accompanied drawings, in which
- Fig. 1: shows a fermenter vessel according to the present invention,
- Fig. 2: is a sectional view of a view port equipped with heating devices for a fermenter vessel according to the present invention,
- Fig. 3: shows a sectional view of the bottom part of a fermenter vessel of the present invention,
- Fig. 4: shows a sectional view of an inlet fitted to a fermenter vessel according to the present invention,
- Fig. 5: shows a top view of an inlet valve according to the present invention, and
- Fig. 6: shows a fermenter system of the present invention comprising more than one fermentation vessel.

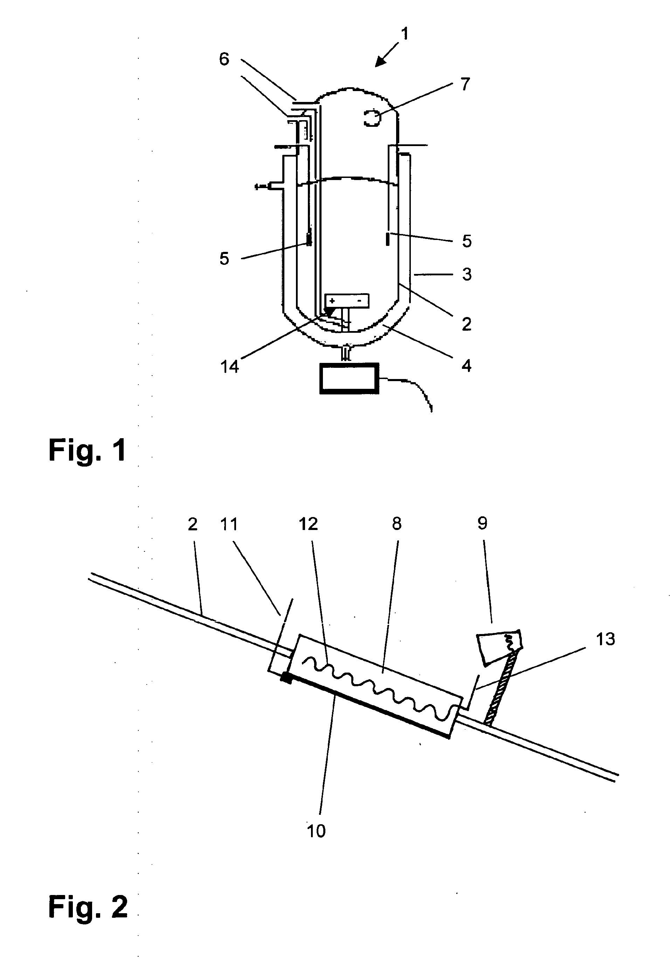

In Fig. 1 a fermenter vessel 1 for biotechnical processing is shown which comprises an outside wall 2. The lower part of the vessel 1 is additionally provided with a second wall 3, so that the lower part has a double jacket wall. A heating medium can be lead through the intermediate space 4 for heating up the broth inside the vessel 1. Furthermore, analyzing probes 5 are provided inside the vessel 1 for measuring relevant parameters like temperature and pressure.

In the upper part of the vessel 1 two inlet ports 6 are arranged through which a gas or liquid can be fed into the vessel 1 and which are connected to a switching valve which is described below. Furthermore, the upper part is provided with a view port 7, with which it is possible to inspect optically the processes inside the vessel 1 without the problem that the broth gets into contact with the outer atmosphere.

The view port 7 is shown in detail in Fig. 2 and it comprises a transparent member 8 with a glass portion and an insulated framing, such as sealed double glazed glass and PVC-U-frames. Alternatively, other materials than glass can be used, such as plastic, or borosilicate glass, quartz, soda-lime-silica glass, or tempered glass.

Because of the increased temperature inside the vessel 1 during processing it is likely that moisture condenses on the transparent member 8. This condensate impedes the view for a user through the transparent member 8. In order to remove the condensate from the surface of the member 8 in this preferred embodiment several heating devices are provided for heating the transparent member 8. These heating devices can be used alternatively or in combination.

As a first means, a heating lamp 9 is arranged outside the vessel 1 adjacent to the view port 7, so that the transparent member 8 can be irradiated by the heating lamp 9 in order to heat up the complete transparent member 8. In this way, it is not necessary to modify the transparent member 8, and common view ports 7 can be used.

As a further heating device preferably the inner surface of the transparent member 8 is provided with a surface heating wire 10 which is connected to a power supply (not shown) via cable 11. With the heating wire 10 on the surface, the transparent member 8 can be efficiently heated up with a small amount of heating energy.

Furthermore, an internal heating wire 12 is incorporated in the transparent member 8 to heat up the complete transparent member 8. Also the internal heating wire 12 can be connected to a power supply (not shown) via line 13.

Due to the heating devices comprising the heating lamp 9 and the heating wires 10, 12 it is possible to reliably remove condensate from the inner surface of the transparent member 8 without the need to provide a mechanical feed through which is necessary in case of a wiper and the sealings of which are often the reason for contaminations.

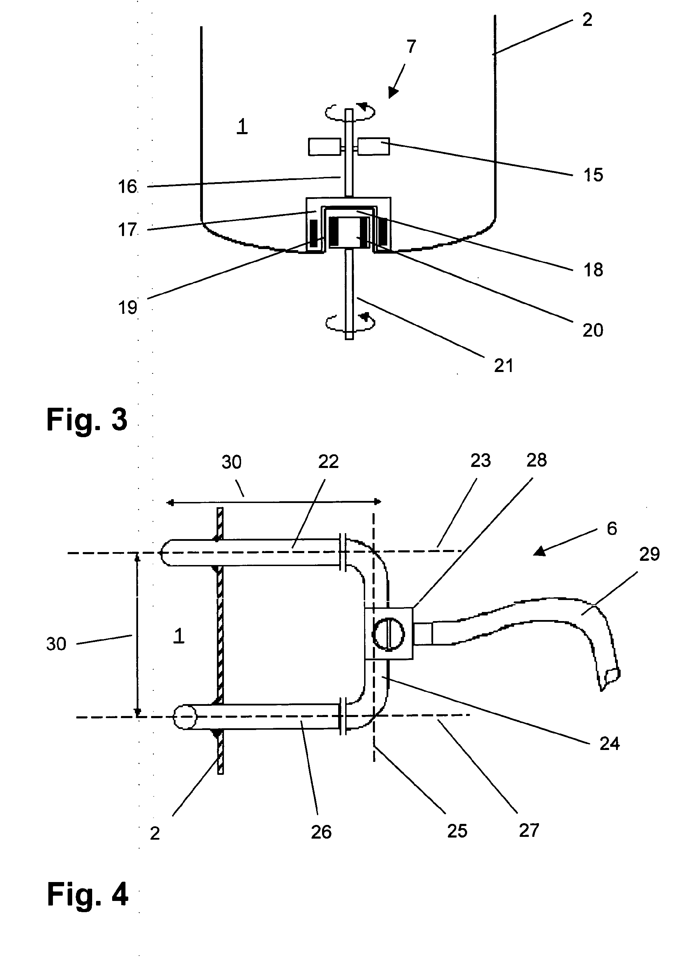

In Fig. 3 a second aspect of the present invention is depicted in detail. A stirring element 14 is arranged in the bottom part of the fermenter vessel 1. It comprises stirring arms 15 which are mounted on a stirrer shaft 16. The stirrer shaft 16 is at its lower end provided with a first magnetic element 17. The outside wall 2 of the vessel 1 has a recess 18 in the bottom part with an annular wall 19 which projects into the vessel 1. The first magnetic element 17 is formed annularly and surrounds the annular wall 19 and is thus arranged adjacent to the recess 18. A second magnetic element 20 is arranged inside the recess 18 and is mounted on a driving shaft 21 which is connected to a motor (not shown) for rotationally driving the driving shaft 21. Furthermore, both the stirrer shaft 16 and the driving shaft 21 are arranged to extend along a common rotational axis.

A non-positive connection between the driving shaft 21 and the stirrer shaft 16 is achieved by the coupling of the magnetic elements 17, 20 via magnetic force. This allows for a rotational drive of the stirring element 14 without a mechanical feedthrough which reduces strongly the risk of contaminations in the fermenter vessel 1 compared to the prior art where a mechanical coupling by a shaft going through the vessel wall was used. The magnetic coupling allows for a complete separation between the stirring element 14 inside the vessel 1 and the driving means outside the vessel 1.

A third aspect of the present invention is depicted in Fig. 4 which shows an inlet 6 for introducing or extracting a gas or liquid into or from the vessel 1. The inlet 6 includes a connection tube to a supply device (not shown) for the gas or liquid wherein the connection tube comprises a first section 22 which extends along a first axis 23 and which is connected with one end to the vessel 1. The first section 22 leads to a second section 24 which extends along a second axis 25. The second axis 25 is essentially perpendicular to the first axis 23. Finally the inlet 6 comprises a third section 26 connected to the second section 24 and extending along a third axis 27 wherein the third axis 27 is essentially parallel and spaced apart to the first axis 23. According to a preferred embodiment, the third section leads back to the vessel 1 which means that a loop is formed by the sections 22, 24, 26. Alternatively, the third section can lead to supply devices, for example, culture medium storage tanks or the like. Furthermore, the second section 24 comprises a junction with an inlet valve 28. A supply tube 29 leading to a media supply (not shown) is connected to the inlet valve 28.

The loop formed by the sections 22, 24, 26 can be bent in the direction of the arrows 30 when the sections 22, 24, 26 expand due to an increase in temperature of the material during sterilization. This ability to expand reduces the tensions in the tubing an thus the risk of crack formation. Furthermore, the loop formed by the three sections 22, 24, 26 allows to scavenge the tube of the inlet 6 between the vessel 1 and the inlet valve 28. Steam can enter the first section 22 and can flow through the second and third sections 24, 26 back to the vessel 1. Thus, the tube between vessel 1 and the inlet valve 28 does not represent a deadleg.

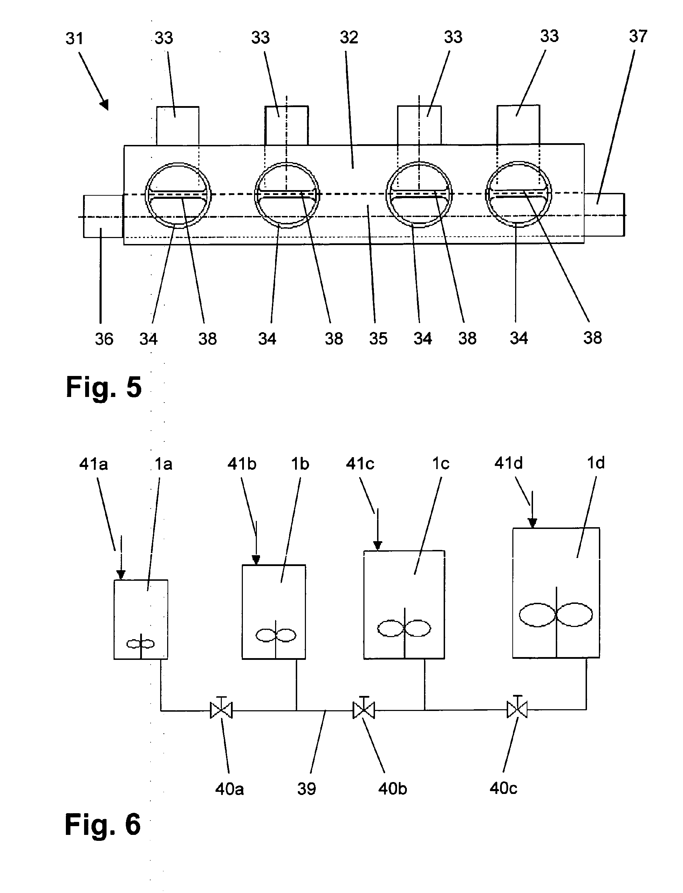

Fig. 5 shows in detail a switching valve 31 used in a fermenter system according to the present invention. The switching valve 31 is utilized to distribute for example cleaning liquids or steam to different positions inside the fermenter vessel. The switching valve 31 is a multiport valve and comprises a valve body 32 which is provided with four outlet studs 33 and with four bores 34 wherein the bores 34 are communicating with the outlet studs 33. Furthermore, in this preferred embodiment the valve body 32 is formed in one piece in order to avoid to weldment joints. A passage tube 35 is passing through the valve body 32 from a first end 36 to a second end 37 and the bores 34 intersect the passage tube 35. An intersection in the sense of the present invention is already present, if part of the wall of the passage tube 35 is intersected by the bores 34 as it is here the case. Both the first end 36 and the second end 37 are communicating with a supply for cleaning liquid (not shown). Thus a loop is formed and the passage tube 35 can be scavenged.

Valve elements 38 are inserted into the bores 34 and can be switched between a closed position and an open position wherein in the open position the passage tube 35 is communicating with the outlet studs 33.

The direct connection between the passage tube 35 and the bores 34 eliminates sections in the tubing which can not be reached with a sufficient flow during sterilization with cleaning liquid which is lead through the passage tube 35. The openings of the passage tube 35 to the bores 34 are directly closed by the valve elements 38 and no space remains which is not scavenged when cleaning liquid is lead through the tubing.

In Fig. 6 a fermenter system is shown which comprises in this preferred embodiment four fermenter vessels 1a, 1b, 1c and 1b. Further, a transfer tube 39 is provided connecting the vessels 1a, 1b, 1c and 1d. The vessels 1a, 1b, 1c and 1d have ports in the bottom section and the transfer tube 39 is connected to these ports. Transfer valves 40a, 40b, and 40c are arranged in the transfer tube 39 between the vessels 1a, 1b, 1c and 1d to allow for the closing of the connection between different vessels 1a, 2b, 1c and 1d.

In this preferred embodiment, the second vessel 1a has a larger volume than the first vessel 1b and the vessel volume increases from the first vessel 1a to the fourth vessel 1d. Further, the vessels 1a, 1b, 1c and 1d are provided with pressure inlets 41a, 41b, 41c and 41d. Through the pressure inlets 41a, 41b, 41c and 41d a pressured medium, preferably pressured air, can be introduced in one of the vessels 1a, 1b, 1c and 1d to increase the pressure in this vessel.

With the transfer tube 39 it is possible that a transfer of broth from the first vessel 1a to the second vessel 1b takes place when the pressure in the first vessel 1a is increased by introducing preferably pressured air via pressure inlet 41a. In this case, the increased pressure in the first vessel 1a forces the broth to move through the transfer tube 39 to the second vessel 1b provided the transfer valve 40a is open and transfer valve 40b is closed. In the same way, broth can be transferred from the second vessel 1b to the third vessel 1c by closing the transfer valves 40a and 40c and by introducing pressured air into the second vessel via pressure inlet 41b. Thus, due to the transfer tube 39, pumps can be omitted and the foam producing effect of the pumps is eliminated. Especially it possible to achieve a "smooth" transfer of culture broth and cells since the pressure increase can be controlled in order to have only a small flow in the transfer tube 39.

The fermenter vessel 1 and the system of the present invention can be used for the culturing of cells, particularly for the culturing of cells for propagation of a virus for the production of a vaccine, more particular a subunit or split vaccine.

More particular still, said virus is a respiratory virus, or an influenza virus (pandemic or annual strain), or poliovirus, or a virus associated with a sexually transmitted disease such as Human Immunodeficiency Virus (HIV), Human Papilloma Virus (HPV), Herpes Simplex Virus (HSV) or Hepatitis C Virus (HCV). More particular still, the cells are animal cells, such as MDCK cells, Vero cells, per.C.6 cells, chick embryo fibroblast (CEF) cells, or CHO cells.

Thus, the present invention also refers to a method for the propagation of cells in which the cells are cultured in a fermenter vessel or a fermenter system according to the accompanied claims. The conditions suitable for the propagation depend on the cells used and are well known to the person skilled in the art. According to the invention, the cells can be animal cells. Preferably, the cells can be MDCK cells, Vero cells, per.C.6 cells, chick embryo fibroblast (CEF) cells, or CHO cells.

According to a further aspect, the invention provides a method for the preparation of a virus vaccine, comprising the step of propagating cells in a fermenter vessel or a fermenter system according to the accompanied claims. Preferably, the method further comprises steps, in which the cells are infected with a virus and subsequently cultured under conditions which allow for the propagation of the virus. Finally, the virus is harvested from the culture broth according to known methods and processed to material which can be used as a basic material for formulating a vaccine. The vaccine can be a subunit or split vaccine. According to the invention, the cells can be animal cells, preferably MDCK cells, Vero cells, per.C.6 cells, chick embryo fibroblast (CEF) cells, or CHO cells. More particular still, said virus is a respiratory virus, or an influenza virus (pandemic or annual strain), or poliovirus, or a virus associated with a sexually transmitted disease such as Human Immunodeficiency Virus (HIV), Human Papilloma Virus (HPV), Herpes Simplex Virus (HSV) or Hepatitis C Virus (HCV).

This method may further comprise steps, wherein one or several components of the virus or the virus are isolated from the cell culture. In a further embodiment the method further comprises the formulation of a vaccine using the component of the virus or the virus obtained from the cell culture. The formulation may comprise a mixing of the component(s) or of the virus with pharmaceutically acceptable carriers, adjuvants and/or excipients. Further, depending on the nature of the vaccination, the virus may have to be inactivated prior to vaccine formulation. A number of methods for virus inactivation are known in the art and may be used for that purpose:

## Claims

1. Fermenter vessel (1) having an outside wall (2) enclosing a vessel volume with a view port (7) being provided in the outside wall (2) for inspecting processes within the vessel volume, the view port (7) having a transparent member (8) wherein the transparent member (8) is provided with a heating device.

2. Fermenter vessel (1) according to claim 1, wherein the heating device comprises a heating lamp (9) arranged outside the vessel volume.

3. Fermenter vessel (1) according to claim 1 or 2, wherein the heating device comprises a heating wire (10) provided on the surface of the transparent member (8).

4. Fermenter vessel (1) according to claim 3, wherein the heating wire (10) is provided on the inner surface of the transparent member (8).

5. Fermenter vessel (1) according to one of the claims 1 to 3, wherein the heating device comprises an internal heating wire (12) which is incorporated in the transparent member (8).

6. Fermenter vessel (1) having an outside wall (2) enclosing a vessel volume with a stirring element (14) arranged inside the vessel volume and with driving means arranged outside the vessel (1) for rotationally driving the stirring element (14) with the stirring element (14) being provided with a first magnetic element (17) and the driving means being provided with a second magnetic (20) element wherein the first magnetic element (17) and the second magnetic element (20) are coupled via magnetic force.

7. Fermenter vessel (1) according to claim 6, wherein the stirring element (14) comprises a stirrer shaft (16) with the first magnetic element (17) being mounted on the stirrer shaft (16).

8. Fermenter vessel (1) according to claim 7, wherein the driving means comprise a driving shaft (21), the second magnetic element (20) being mounted on the driving shaft (21) .

9. Fermenter vessel (1) according to claim 8, wherein the stirrer shaft (16) and the driving shaft (21) extend along a rotational axis.

10. Fermenter vessel (1) according to one of the claims 6 to 9, wherein the vessel (1) has a recess (18) in the outside wall (2), the recess (18) having an annular (19) wall projecting into the vessel volume wherein the second magnetic element (20) is arranged inside the recess (18).

11. Fermenter vessel (1) according to claim 10, wherein the first magnetic element (17) is arranged adjacent to the annular wall (19).

12. Fermenter vessel (1) according to one of the claims 6 to 11, wherein the vessel (1) further comprises the features specified in any of claims 1 to 5.

13. Fermenter vessel (1) having an outside wall (2) enclosing a vessel volume, the outside wall (2) comprising an inlet (6) for introducing or extracting a gas or liquid into or from the vessel volume, and a connection tube to a supply device for the gas or liquid wherein the connection tube comprises a first section (22) extending along a first axis (23), a second section (24) connected to the first section (22) and extending along a second axis (25), the second axis (25) being essentially perpendicular to the first axis (23), and a third section (26) connected to the second section (24) and extending along a third axis (27), the third axis (27) being parallel and spaced apart to the first axis (23).

14. Fermenter vessel (1) according to claim 13, wherein the second section comprises a junction.

15. Fermenter vessel (1) according to claim 14, wherein the junction compises an inlet valve (28).

16. Fermenter vessel (1) according to one of the claims 13 to 15, wherein the third section (26) is connected the vessel volume.

17. Fermenter vessel (1) according to one of the claims 13 to 16, wherein the vessel (1) further comprises the features specified in any of claims 1 to 5.

18. Fermenter vessel (1) according to one of the claims 13 to 17, wherein the vessel (1) further comprises the features specified in any of claims 6 to 11.

19. Fermenter system with a vessel (1) enclosing a vessel volume and with a tubing for distributing liquids in the fermenter system wherein the tubing comprises a switching valve (31), the switching valve having a valve body (32), a plurality of outlet studs (33), a passage tube (35) passing through the valve body (32) from a first end (36) to a second end (37), a plurality of bores (34) in the valve body (32) and a plurality of valve elements (38) inserted into the bores (34) wherein the first end (36) and the second end (37) are communicating with a liquid supply, wherein the bores (34) are communicating with the outlet studs (33) and wherein the bores (34) intersect the passage tube (35).

20. Fermenter system according to claim 19, wherein the valve body (32) is formed in one piece.

21. Fermenter system according to claim 19 or 20, wherein the system comprises a fermenter vessel (1) according to any of claims 1 to 18.

22. Fermenter system comprising a first fermenter vessel (1a), a second fermenter vessel (1b) and a transfer tube (39) connecting the first vessel (1a) and the second vessel (1b), wherein the transfer tube (39) is connected to the bottom section of the first vessel (1a) and to the bottom section of the second vessel (1b).

23. Fermenter system according to claim 22, wherein a transfer valve (40a) is provided in the transfer tube (39).

24. Fermenter system according to claim 22 or 23, wherein the first vessel (1a) is provided with a pressure inlet (41a) for introducing a pressured medium into the first vessel (1a).

25. Fermenter system according to one of the claims 22 to 24, wherein the second vessel (1b) has a larger volume than the first vessel (1a).

26. Fermenter system according to any of claims 22 to 25, wherein the system comprises a fermenter vessel (1) according to any of claims 1 to 18.

27. Fermenter system according to any of claims 22 to 26, wherein the system further comprises the features specified in claim in 19 or 20.

28. Method for the propagation of cells, comprising the step of culturing the cells in a fermenter vessel according to claims 1 to 18 or a fermenter system according to claims 19 to 27.

29. Method for the preparation of a virus vaccine, comprising the step of propagating cells in a fermenter vessel according to claims 1 to 18 or a fermenter system according to claims 19 to 27.

30. Method according to claims 28 or 29, wherein said cells are animal cells.

31. Method according to claim 30, wherein said cells are selected from group consisting of MDCK cells, Vero cells, per.C.6 cells, chick embryo fibroblast (CEF) cells, or CHO cells.

32. Method according to one of claims 29 to 31, wherein the method further comprises isolating a component of the virus or the virus from the cell culture.

33. Method according to claim 32, wherein the method further comprises the formulation of a vaccine using the component of the virus or the virus obtained from the cell culture.
